# EUROPÄISCHE PATENTSCHRIFT

(11) **EP 2 120 775 B1**
(45) Veröffentlichungstag und Bekanntmachung des Hinweises auf die Patenterteilung: **29.06.2016**
(21) Anmeldenummer: 08706966.2
(22) Anmeldetag: 08.01.2008
(51) Int. Cl.: A61C 1/08, A61B 17/17

(54) **ANORDNUNG MIT EINEM INSTRUMENT ZUR VORBEREITUNG ODER DURCHFÜHRUNG DER INSERTION EINES IMPLANTATS**
ARRANGEMENT COMPRISING AN INSTRUMENT FOR THE PREPARATION OR EXECUTION OF THE INSERTION OF AN IMPLANT
SYSTÈME COMPRENANT UN INSTRUMENT POUR LA PRÉPARATION OU LA MISE EN OEUVRE DE L'INSERTION D'UN IMPLANT

(30) Priorität: 26.01.2007 DE 102007004948
(43) Veröffentlichungstag der Anmeldung: 25.11.2009
(73) Patentinhaber: DENTSPLY Implants Manufacturing GmbH, 68229 Mannheim (DE)
(72) Erfinder: DREWS, Dirk-Joachim, 69181 Leimen (DE); OBERMEIER, Frank, 64646 Heppenheim (DE); WOLF, Dietrich, 73447 Oberkochen (DE)
(74) Vertreter: Schmitt, Meinrad
(86) Internationale Anmeldenummer: PCT/EP2008/000067
(87) Internationale Veröffentlichungsnummer: WO 2008/089885

(56) Entgegenhaltungen:
- WO-A-02/100290
- US-A- 5 888 034
- US-A- 5 967 777
- US-A1- 2005 170 311
- US-A1- 2006 260 110

## Beschreibung

Die Erfindung bezieht sich auf eine Anordnung zur Vorbereitung oder Durchführung der Insertion eines Implantats, insbesondere eines Dentalimplantats, gemäß den im Oberbegriff des Patentanspruchs 1 angegebenen Merkmalen.

Eine derartige Anordnung ist aus der US 5 320 529 bekannt und enthält ein als Bohrer ausgebildetes Instrument und eine Schablone, mittels welcher die Positionierung und Einbringung einer Bohrung in einem Knochen, insbesondere Kieferknochen, vorgebbar ist. Die Schablone, welche auch als Schiene bezeichnet wird oder als solche ausgebildet ist, wird aufgrund von Daten hergestellt, welche mittels eines Computertomographen (CT), eines Röntgengerätes oder einer sonstigen bilderzeugenden Einrichtung des Knochens, insbesondere des Kieferknochens, erzeugt werden, um die erforderliche Position des Implantats unter Berücksichtigung der anatomischen, chirurgischen und auch der ästhetischen Gegebenheiten planen und definiert vorgeben zu können. Die Schiene ist auf die jeweilige Situation und Gegebenheiten angepasst und enthält wenigstens eine durchgehende Ausnehmung zur Aufnahme einer ersten Hülse. Diese erste Hülse enthält eine Durchgangsbohrung, durch welche das als Bohrer ausgebildete Instrument durchführbar ist, um eine exakte Positionierung bzw. Ausrichtung der Bohrung für das Implantat vorgeben zu können. Der Innendurchmesser der Durchgangsbohrung der genannten Hülse ist auf den Außendurchmesser des Bohrers zum Einbringen der Bohrung in den Knochen abgestimmt. Der Bohrer enthält einen Ringbund oder Anschlag, welcher auf der freien Oberkante der Hülse zur Anlage bringbar ist, um somit die Tiefe der Bohrung im Knochen vorzugeben. Beim Einbringen eines Implantats in die mittels der Anordung eingebrachte Bohrung, welche auch als aufbereitetes Implantatbett bezeichnet wird, ergeben sich in der Praxis Probleme, vor allem dahingehend, dass die Einbringtiefe des Implantats vom Operateur nur sehr schwer kontrollierbar ist. Des Weiteren können beim Einführen des Bohrers in die Hülse Schäden am Bohrer und/ oder der Innenfläche der Hülse auftreten, insbesondere infolge einer nicht exakten koaxialen Anfangsausrichtung des Bohrers bezüglich der Durchgangsbohrung der Hülse. Der Innendurchmesser der Durchgangsbohrung der Hülse ist zumindest gleich groß wie der Außendurchmesser des Bohrers, welcher nach dem Einführen in die Durchgangsbohrung nach Art einer Schiebepassung in dieser bewegbar und/oder drehbar ist. Bei einem zu großen Spiel bzw. Durchmesserunterschied zwischen Außendurchmesser des Bohrers und Innendurchmesser der Durchgangsbohrung ist eine exakte Ausrichtung und Einbringung der Bohrung in Frage gestellt. Des Weiteren besteht die Gefahr, dass sobald die Anlagefläche des Bohrers an die Anlagefläche der Hülse zur Anlage gelangt und die Drehung des Bohrers nicht sofort beendet wird, die Hülse sich infolge der auftretenden Reibungsverluste erheblich erwärmt. Infolgedessen ist der feste Sitz der Hülse in der üblicherweise aus Kunststoff bestehenden Schablone nicht mehr gewährleistet mit dem Risiko, dass der Bohrer die in dem Knochen bereits eingebrachte Bohrung aufweitet und/oder in ihrer Ausrichtung in unzulässiger Weise verändert.

Eine andere derartige Anordnung ist aus der US5967777 bekannt.

Weiterhin ist aus der DE 22 05 314 C3 eine Vorrichtung zur Infusion von flüssigen Mitteln in die Knochen bekannt, enthaltend eine äußere Röhre, deren in das Knochengewebe einzuführendes Ende ein Außengewinde aufweist. Die Vorrichtung enthält ferner eine innere Röhre mit einer geschärften Spitze mit Öffnungen für die Injektion sowie am entgegengesetzten Ende eine Kanüle. An der äußeren Hülse ist ein Begrenzer vorgesehen und oberhalb der durchstochenen Haut in eine Lage feststellbar, wobei die Befestigung mittels einer Schraube erfolgt. Der als Hülse ausgebildete Begrenzer ermöglicht allein die Begrenzung der Einbringtiefe der Vorrichtung in den Knochen. Hiervon ausgehend liegt der Erfindung die Aufgabe zugrunde, die aufgezeigten Schwierigkeiten zu vermeiden und die Anordnung dahingehend weiterzubilden, dass eine exakte Ausrichtung und/oder Führung des Instruments und/oder Handhabung bei der Durchführung der Arbeiten mit dem Instrument und/oder bei der Insertion des Implantats in das aufbereitete Implantatbett oder insbesondere dem Ausstanzen der Schleimhaut und/oder Einbringung der Bohrung in den Knochen mit hoher Sicherheit erreicht werden. Die Anordnung soll einen einfachen Aufbau aufweisen und insbesondere soll die Schablone in Kombination mit unterschiedlichen Instrumenten wie Stanze, Bohrer, Gewindeschneider oder Eindrehwerkzeug zum Einsatz gelangen. Die Anordnung soll einen einfachen und gleichwohl funktionssicheren Aufbau aufweisen und/oder eine problemlose, zuverlässige Handhabung bei Durchführung von Bearbeitungsvorgängen mittels des Instruments ermöglichen.

Die Lösung dieser Aufgabe erfolgt gemäß den im Patentanspruch 1 angegebenen Merkmalen.

Die erfindungsgemäße Anordnung zeichnet sich durch einen funktionsgerechten und einfachen Aufbau aus und ermöglicht in einfacher Weise problemlos die exakte Ausrichtung und/oder Führung und/oder Positionierung des Instruments bezüglich des Knochens und/oder der außen auf dem Knochen vorhandenen Schleimhaut. Das Instrument kann für unterschiedliche Arbeitsvorgänge ausgebildet sein, wobei hier vor allem auf eine Stanze für die Aufbereitung der Schleimhaut im Bereich des einzubringenden Implantats, einen Bohrer, einen Gewindeschneider zur Erzeugung eines Innengewindes im Implantatbett, und ferner auf ein Eindrehwerkzeug verwiesen sei. Die nachfolgenden Erläuterungen, konstruktiven Merkmale, Vorteile und Wirkungszusammenhänge bei Ausbildung des Instruments als Bohrer gelten analog auch für in anderer Weise ausgebildete Instrumente. Weiterhin sei festgehalten, dass im Rahmen der Erfindung die für das Hindurchführen des Instruments vorgesehene Ausnehmung bzw. Durchgangsbohrung entweder unmittelbar in der Schablone oder mittelbar in einer in die Schablone integrierten ersten Hülse vorhanden sein kann, wobei die Ausnehmung in der ersten Hülse weiterhin als Durchgangsbohrung bezeichnet wird. Auf dem Instrument ist eine zweite Hülse angeordnet, deren Außenkontur an die Innenkontur der Durchgangsbohrung, insbesondere der ersten mit der Schablone verbundenen Hülse abgestimmt ist. Das Instrument ist nicht direkt in der Durchgangsbohrung der ersten Hülse oder Schablone geführt, sondern mittelbar mittels der zweiten Hülse oder Verschiebehülse. In der Grundposition der zweiten Hülse ragt die Spitze des Instruments bevorzugt um einen vorgegebener Länge aus der zweiten Hülse verhaus, und das Instrument kann somit zunächst auch nicht exakt koaxial in die genannte Durchgangsbohrung eingeführt werden, und zwar so weit, bis die zweite Hülse mit ihrem der Spitze des Instruments zugewandten Ende in die Durchgangsbohrung eingreift. Die zweite Hülse ist auf und/oder bezüglich des Instruments mittels lösbaren Halteelementen und/oder Rastelementen in einer Grundposition axial derart festgelegt, dass nach Überschreiten einer vorgegebenen axialen Haltekraft die zweite Hülse selbsttätig in axialer Richtung auf dem Instrument verschiebbar ist, und zwar insbesondere in axialer Richtung weg vom freien Ende des Instruments, welches nachfolgend in die den Knochen umgebende Schleimhaut und/oder in den Knochen einführbar ist. Die Halte- und/oder Rastelemente sind auf dem Instrument bevorzugt in einem Bereich außerhalb von Bearbeitungsflächen, wie Schneidflächen eines Bohrers oder dergleichen, angeordnet, wobei derartige Bearbeitungsflächen sich zwischen der Spitze oder dem freien Ende des Instruments und dem das Halte- und/oder das Rastelement aufweisenden Bereich befinden. Nach dem selbsttätigen Freigeben und/oder Lösen der Halte- und/oder Rastelemente des Instruments einerseits und der zweiten Hülse, welche nachfolgend auch als Führungshülse bezeichnet wird, ist diese auf dem Instrument in axialer Richtung verschiebbar, insbesondere bis zu einem vorgegebenen Anschlag. Der Schaft des Instruments ist zwischen dem Bereich, in welchem die Verschiebehülse sich in der Grundposition befindet und dem Anschlag in bevorzugter Weise zylindrisch und/oder glatt und/oder als Führungsfläche für die Verschiebehülse und/oder deren Innenfläche ausgebildet, zwecks sicherer Führung der Verschiebehülse nach deren automatischer Freigabe. Die Außenkontur der zweiten Hülse ist vorteilhaft im Wesentlichen zylindrisch und/oder ohne Gewinde, somit glatt ausgebildet, und analog hierzu die Innenfläche der Durchgangsbohrung und/oder der ersten Hülse.

Vorteilhaft weisen die Innenfläche der ersten Hülse und die zugeordnete Außenfläche der zweiten Hülse glatte und/oder polierte Oberflächenstrukturen auf. Der Innendurchmesser der Durchgangsbohrung und/oder der ersten Hülse ist wesentlich größer als der Außendurchmesser des Instruments, welcher in der zweiten Hülse geführt wird. Die Durchmesserdifferenz der Innenfläche der ersten Hülse zum Außendurchmesser des Instruments ist im Wesentlichen gleich groß wie die doppelte Wanddicke der zweiten Hülse. Beim Einsetzen des Instruments in die Durchgangsbohrung der Schablone bzw. der ersten Hülse ist infolge der genannten Durchmesserdifferenz die Gefahr einer Beschädigung der ersten Hülse weitestgehend ausgeschlossen. Die zweite Hülse ist auf dem Instrument mittels lösbaren Verbindungselementen festgelegt. Die automatisch und/oder selbsttätig lösbare Verbindung ist derart angeordnet und/oder ausgebildet, dass beim Erreichen einer vorgegebenen axialen Kraft die Verbindung selbsttätig gelöst wird und die Führungshülse nachfolgend in axialer Richtung auf dem Instrument verschiebbar ist. Die Halteelemente sind bevorzugt als miteinander korrespondierende und in Eingriff stehende aber gleichwohl bei Erreichen der vorgegebenen Haltekraft selbsttätig lösbare Rastelemente. Weiterhin sind das Instrument und die Hülse bzw. Führungshülse derart aufeinander abgestimmt, dass nach dem Lösen und/oder Freigeben der Verbindung das Instrument in und/oder mittels der Hülse axial geführt wird und somit eine präzise Handhabung des Instruments erreicht wird, beispielsweise beim weiteren oder tieferen Einbringen eines Bohrers in den Knochen. Das Instrument wird exakt geführt, wobei die zweite Hülse bezüglich der Schablone, insbesondere durch aufeinander abgestimmte Anschläge, bezüglich der Schablone in axialer und/oder radialer Richtung arretiert ist. Die zweite Hülse oder verschiebbare Hülse weist eine Anlagefläche auf, welcher eine Anschlagfläche der Schablone oder der ersten Hülse derart zugeordnet ist, dass bei Anlage der genannten Flächen und Einwirken der genannten axialen Kraft die Verbindungselemente automatisch gelöst bzw. freigegeben werden.

Die zweite Hülse ist in vorteilhafter Weise auf dem Instrument derart lösbar festgelegt, dass, bevor die Spitze des Instruments den Knochen erreicht, die zweite Hülse zumindest teilweise in die erste Hülse bereits eingeführt ist und somit eine sichere Ausrichtung des Instruments, beispielsweise des Bohrers, schon zu Beginn des Anbohrens des Knochens bezüglich diesem exakt ausgerichtet ist. Beim Weiterbohren gelangt dann die zweite Hülse mit einem Anschlag an einen zugeordneten Anschlag der ersten Hülse zur Anlage, so dass nach weiterem Einwirken einer axialen Kraft in Längsrichtung zur Bohrerspitze hin die lösbare Verbindung der zweiten Hülse auf dem Bohrer automatisch freigegeben wird und der Bohrer nunmehr durch die zweite Hülse zum weiteren Aufbohren des Knochens geschoben werden kann. Es wird somit eine funktionssichere und/oder achsparallele und/oder koaxiale Führung des Instruments gewährleistet. Das Einbringen der Bohrung wird beendet, sobald ein Anschlag des Bohrers mittelbar über die zweite Hülse mit der in der Schablone festgelegten ersten Hülse zur Anlage gelangt, wodurch ferner die Einbringtiefe definiert vorgegeben ist. Es sei hier ausdrücklich darauf hingewiesen, dass die vorstehenden Erläuterungen analog auch dann gelten, wenn anstelle des Bohrers ein anderes Instrument, wie insbesondere eine Stanze für die Schleimhaut, ein Gewindeschneider oder ein Eindrehwerkzeug für das Implantat zum Einsatz gelangen. Die vorgeschlagene Anordnung gelangt erfindungsgemäß zur Verwendung bei der Vorbereitung oder Durchführung der Insertion eines Implantats und ist ferner erfindungsgemäß für die Durchführung eines Verfahrens zur Vorbereitung oder Insertion eines Implantats ausgebildet.

Weiterbildungen und besondere Ausgestaltungen der Erfindung sind in den Unteransprüchen und der nachfolgenden Beschreibung angegeben.

Die Erfindung wird nachfolgend anhand der in der Zeichnung dargestellten besonderen Ausführungsbeispiele näher erläutert, ohne dass insoweit eine Beschränkung erfolgt. Es zeigen:
- Fig. 1: teilweise geschnitten die Anordnung in perspektivischer Darstellung, wobei das Instrument als Bohrer ausgebildet ist,
- Fig. 2: einen Schnitt der zweiten verschiebbaren Hülse in einer Axialebene,
- Fig. 3, 4: die erste Hülse in einem axialen Schnitt und in perspektivischer Darstellung,
- Fig. 5, 6: die Anordnung in unterschiedlichen Positionen des Bohrers während des Einbringens der Bohrung in den Knochen,
- Fig. 7, 8: perspektivische Darstellungen des Bohrers mit der verschiebbaren Hülse,
- Fig. 9, 10: besondere Ausbildungen der lösbaren Verbindung der zweiten Hülse mit dem Bohrer,
- Fig. 11, 12: ein besonderes Ausführungsbeispiel der lösbaren Verbindung der zweiten Hülse mit dem Schaft des Bohrers,
- Fig. 13: eine Ansicht einer besonderen Schaftgeometrie.

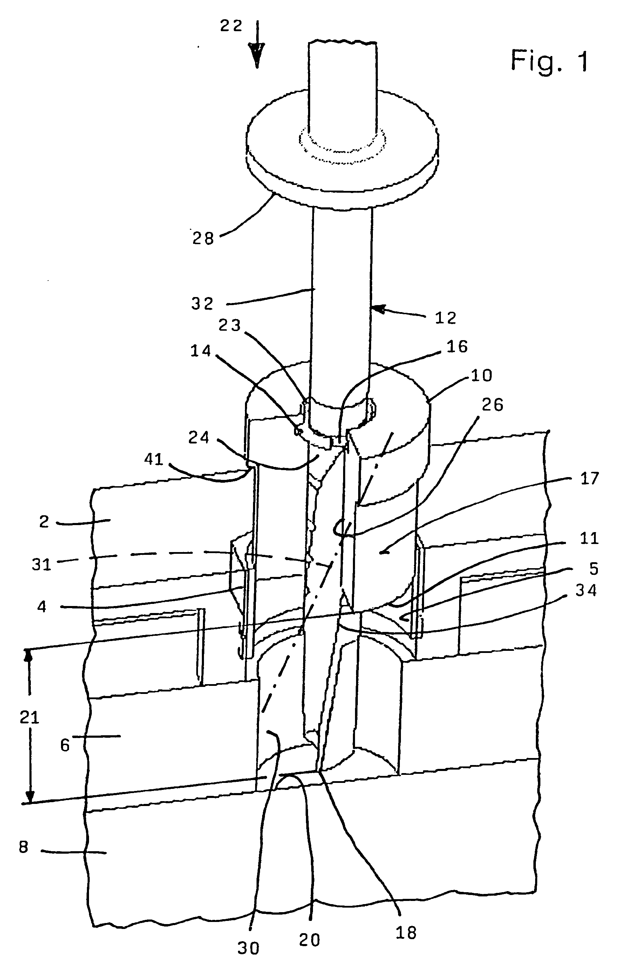

Gemäß Fig. 1 enthält die erfindungsgemäße Anordnung eine Schablone 2, in welcher eine erste Hülse 4 angeordnet und insbesondere aufgrund einer nicht runden Außenfläche drehgesichert ist. Die Schablone 2 und/oder die erste Hülse 4 enthalten eine durchgehende Ausnehmung 5. Die Schablone 2 mit der bevorzugt integrierten ersten Hülse 4 ist auf eine Schleimhaut bzw. Mucosa 6 aufgelegt, welche sich über einem Knochen 8, insbesondere Kieferknochen, befindet. In die erste Hülse 4 bzw. die Ausnehmung 5 ist eine zweite Hülse 10 mit dem unteren Ende 11 bereits teilweise eingeführt, welche auf einem Instrument in Form eines Bohrers 12 angeordnet ist. Die zweite Hülse ist mittels automatisch lösbaren Verbindungselementen 14, 16 auf dem Instrument bzw. Bohrer 12 festgelegt. Die Verbindungselemente 14, 16 sind erfindungsgemäß als ineinander greifende Rastelemente ausgebildet, welche beim Überschreiten einer vorgegebenen axialen Kraft außer Eingriff gebracht werden, so dass die Hülse 10 nachfolgend auf dem hier als Bohrer 12 ausgebildeten Instrument axial verschoben wird. Die zweite Hülse 10 weist eine Außenfläche 17 auf, welche auf die Innenfläche der Ausnehmung 5 abgestimmt und/oder an diese angepaßt ist. Bevorzugt sind die Außenfläche 17 und die genannte Innenfläche der Ausnehmung 5 zylindrisch derart ausgebildet, dass die zweite Hülse 10 im Wesentlichen spielfrei in der Ausnehmung 5 drehbar und/oder axial verschiebbar ist.

Nachfolgend wird der Einfachheit halber das Instrument als Bohrer 12 bezeichnet, wobei im Rahmen der Erfindung das Instrument insbesondere als Stanze, Gewindeschneider oder Eindrehwerkzeug des Implantats ausgebildet sein kann. Die Festlegung der zweiten Hülse 10 auf dem Bohrer 12 ist in bevorzugter Weise derart vorgegeben, dass die zweite Hülse 10, wie dargestellt, zumindest teilweise in die erste Hülse 4 bzw. Die Ausnehmung 5 eingeführt ist, bevor die Bohrerspitze 18 die Oberfläche 20 des Knochens 8 erreicht. Die Spitze 18 steht über das untere Ende 10 der zweiten Hülse oder Verschiebehülse 10 in deren Grundposition in der Länge 21 heraus. Das erste lösbare Verbindungselement 14 ist vorteilhaft als ein elastischer Ring und/oder ein aufweitbares Federelement ausgebildet und teilweise in einer Ringnut in der Innenfläche der zweiten Hülse 10 angeordnet. Der Bohrer 12 enthält in seiner Außenfläche das zweite lösbare Verbindungselement 16, welches hier als eine Ringnut ausgebildet ist, in welcher das korrespondierend ausgebildete erste Verbindungselement 14 der zweiten Hülse 10 teilweise eingreift. Die lösbare Verbindung bzw. deren miteinander korrespondierende Verbindungselemente 14, 16 sind derart ausgebildet, dass beim Überschreiten einer vorgebbaren axialen Kraft gemäß Pfeil 22 zwischen Bohrer 12 und zweiter Hülse 10 die Verbindung freigegeben wird. Wie ersichtlich, enthält die zweite Hülse 10 eine Durchgangsbohrung 23, durch welche der Bohrer 12 durchgreift. Der Bohrer 12 bzw. dessen Außenfläche 24 ist auf die zweite Hülse 10 bzw. deren Innenfläche 26 derart abgestimmt, dass nach dem automatischen Freigeben der lösbaren Verbindung 14, 16 die zweite Hülse 10 auf dem Bohrer 12 verschiebbar ist. Die zweite Hülse 10 kann somit auch als Verschiebehülse bezeichnet werden, welche in axialer Richtung auf der Außenfläche des Bohrers axial verschiebbar ist, wobei ferner der Bohrer 12 drehbar in der Verschiebehülse 10 angeordnet ist. Es sei ausdrücklich darauf hingewiesen, dass der Bohrer 12 bevorzugt auch dann in der Verschiebehülse 10 drehbar ist, wenn die lösbare Verbindung 14, 16 zwischen der Verschiebehülse 10 und dem Bohrer 12 nicht freigegeben ist. Mittels der lösbaren Verbindung 14, 16 ist die Verschiebehülse 10 auf dem Bohrer 12 axial festgelegt, wobei gleichwohl die Rotation des Bohrers 12 in der Verschiebehülse 10 ermöglicht ist. Der Bohrer 12 enthält ferner einen Anschlag 28, welcher hier als ein Ringbund ausgebildet ist. In der dargestellten und mittels der Verbindungselemente 15, 16 gesicherten Grundposition der Verschiebehülse 10 ist zwischen dieser und dem Anschlag 28 ein vorgegebener axialer Abstand vorhanden, mit welchem die Einbringtiefe des Bohrers 12, bzw. der Bohrerspitze 18 in den Knochen 8 definiert vorgegeben ist.

Vor dem Einführen des Bohrers mit der Verschiebehülse 10 in die Schablone 2 wurde in die Schleimhaut oder Mucosa 6 eine Öffnung 30 mittels eines geeigneten Instruments, beispielsweise einem Stanzwerkzeug, eingebracht. Auch das genannte Stanzwerkzeug ist derart ausgebildet und/oder an die Schablone 2 und/oder deren erste Hülse 4 angepasst, dass eine sichere Ausrichtung und Führung vorgegeben ist. Das genannte Stanzwerkzeug ist ebenfalls Bestandteil der erfindungsgemäßen Anordnung Ferner sei angemerkt, dass der Bohrer bzw. das Instrument 12 zunächst, wie mit strichpunktierter Linie 31 angedeutet, schräg mit seiner Spitze 18 in die erste Hülse 4 eingreifend soweit eingeführt werden kann, bis das Ende 21 der Verschiebehülse 10 mit der ersten Hülse 4 in Eingriff gebracht ist und somit die koaxiale Ausrichtung des Instruments 12 mit der Durchgangsbohrung erreicht ist.

Zusätzlich oder alternativ kann die Verschiebehülse 10, anstelle einer geschlossenen ringförmigen Ausbildung ferner einen axialen Schlitz derart aufweisen, dass die Verschiebhülse 10 seitlich auf den Bohrer 12 aufgeschoben und/oder aufgeklippst werden kann. Hierbei besteht die Verschiebehülse 10 aus einem vorteilhaft federelastischen Werkstoff, insbesondere aus Kunststoff. Eine derartige Ausbildung entspricht im Wesentlichen der in Fig. 1 geschnitten dargestellten Ausbildung der Verschiebehülse 10. Des Weiteren kann die Festlegung der Verschiebehülse 10 am im Wesentlichen zylindrischen Bohrerschaft 32 oder im Schneidbereich 34 oder am hier nicht dargestellten Bohrfutter erfolgen, wobei im letzten Fall selbstverständlich der Anschlag 28 an einer anderen Stelle angeordnet ist. Weiterhin kann im Rahmen der Erfindung der Anschlag als Bestandteil des Bohrfutters oder durch ein korrespondierendes Bauelement des Bohrgerätes ausgebildet sein. Unabhängig von der konkreten Ausgestaltung der lösbaren Verbindungselemente 14, 16 und deren räumliche Anordnung auf und/oder an dem Bohrer und/oder deren räumliche Zuordnung zum Bohrer kommt es maßgeblich darauf an, dass die Verschiebehülse 10 zunächst bzw. im werksseitig vorgegebenen Auslieferungszustand bzw. in der Grundposition in definierter Weise axial auf dem Bohrer mittels lösbarer Verbindungselemente festgelegt ist und dass ferner nach dem Lösen oder Freigeben der Verbindungselemente eine definierte axiale Führung und/oder Verschiebung ermöglicht wird, welche nach Zurücklegung eines definiert vorgegeben Verschiebeweges mittels des Anschlags 28 begrenzt ist.

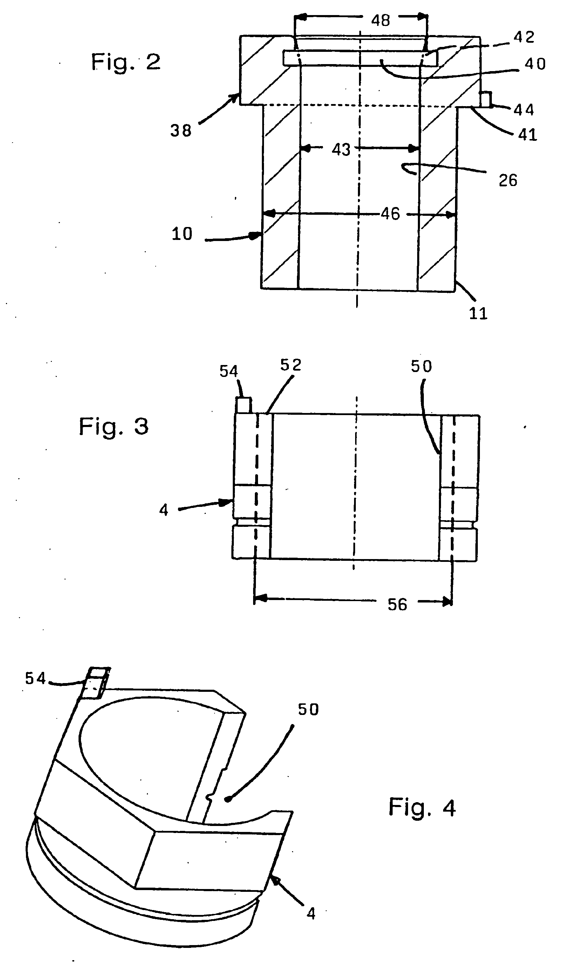

Fig. 2 zeigt einen Schnitt durch die Verschiebehülse 10, welche in ihrer Innenfläche 26, bevorzugt deren oberen Endbereich 38, eine Ringnut 40 zur teilweisen Aufnahme des ersten lösbaren Verbindungselements enthält. Die untere bzw. der Spitze des Instruments zugewandte Endfläche 41 des als Ringbund mit erweitertem Durchmesser ausgebildeten Endbereichs 38 bildet die Anlagefläche der Verschiebehülse 10 an der Schablone und/oder der in diese integrierten ersten Hülse. Gelangt die untere Endfläche 41 an der Schablone und/oder der integrierten ersten Hülse zur Anlage und ist die axiale Vorschubkraft weiterhin wirksam, so wird die Verbindung der Verschiebehülse 10 mit dem Bohrer bzw. Instrument automatisch und/oder ohne zusätzliche Maßnahmen gelöst. Wie mit strichpunktierter Linie 42 angedeutet, kann im Rahmen der Erfindung das erste lösbare Verbindungselement ferner als ein konischer Flächenteil ausgebildet sein oder einen solchen enthalten, welcher mit einem korrespondierend ausgebildeten Flächenteil des Instruments, insbesondere Bohrers, zusammenwirkt, wobei vorteilhaft auch eine Drehsicherung hierdurch realisiert ist. Der bzw. die Konuswinkel sind derart vorgegeben, dass keine konische Selbsthemmung erfolgt, sondern vielmehr die lösbare Verbindung vorgegeben ist. Die Verschiebehülse 10 bzw. deren Innenfläche 26 besitzt, ausgehend von der Ringnut 40 bis zum unteren Ende 11, einen gleichbleibenden Innendurchmesser 43. Es sei festgehalten, dass die erfindungsgemäße Anordnung verschiedene Verschiebehülsen 10 enthält, und zwar mit unterschiedlich großen Innendurchmessern 43, welche auf unterschiedlich große Außendurchmesser verschiedener Bohrer bzw. Instrumente abgestimmt sind. Entsprechend ausgebildet und/oder abgestimmt sind bei solchen Verschiebehülsen ferner der Innendurchmesser der Ringnuten 40 sowie der in diesen angeordneten Verbindungselemente.

Ferner ist außen an der Verschiebehülse 10 ein Anschlagelement 44 vorgesehen, mit welchem ein weiteres Anschlagelement der ersten Hülse und/oder der Schablone derart korrespondiert, dass, sobald diese miteinander in Eingriff und/oder Anlage gelangen, die zunächst vorgegebene Drehung gestoppt wird und die Verschiebehülse bezüglich der Schablone und/oder der ersten Hülse drehgesichert ist. Die Verschiebehülse 10 besitzt, abgesehen vom oberen erweiterten Endbereich 38, einen Außendurchmesser 46, welcher auf den Innendurchmesser der ersten Hülse abgestimmt ist, und zwar bevorzugt derart, dass eine Schiebepassung vorliegt und/oder dass die Verschiebehülse 10 nach dem Eingreifen in die erste Hülse in dieser leicht gängig und/oder unter Vermeidung eines Spiels bewegbar ist, und zwar sowohl axial als auch drehend. Es sei ausdrücklich festgehalten, dass die Verschiebehülsen mit unterschiedlichen Innendurchmessern 43 jeweils die gleichen Außendurchmesser 46 aufweisen. Oberhalb der Ringnut 40 für das Verbindungselement weist die Verschiebehülse 10 einen Innendurchmesser 48 auf, welcher in bevorzugter Weise größer vorgegeben ist als der Innendurchmesser 43 der von der Ringnut 40 zum unteren Ende 11 hin anschließenden Innenfläche 26. Somit wird in bevorzugter Weise sichergestellt, dass bei und/oder nach dem Lösen der Verbindung mit dem Instrument das in der Ringnut 40 angeordnete Verbindungselement nicht nach unten in den Bereich der Innenfläche 36 gepreßt wird, sondern nach oben in den Bereich mit dem erweiterten Durchmesser 48 teilweise ausweichen und/oder verdrängt werden kann. Wie ersichtlich, ist der Innendurchmesser der Ringnut 40 größer vorgegeben als der Innendurchmesser 48 des nach oben anschließenden Bereichs.

In Fig. 3 und 4 ist die erste Hülse 4 in einer seitlichen Ansicht und perspektivisch dargestellt. Die erste Hülse 4 enthält einen seitlichen Schlitz 50, welcher sich von der oberen Stirnfläche 52 zumindest teilweise nach unten erstreckt, bevorzugt gemäß Zeichnung über die gesamte axiale Länge. Die erste Hülse 4 weist eine zumindest teilweise unrunde Außenkontur, beispielsweise in Form eines Mehrkants auf, zwecks drehfester Integration in die Schablone. Des Weiteren enthält auch die Schablone eine mit dem seitlichen Schlitz 50 der Hülse 4 korrespondierende Ausnehmung oder einen Schlitz derart, dass das Instrument bzw. dessen Spitze seitlich, ggf. schräg, in die Schablone und/oder die erste Hülse eingeführt werden kann. Entsprechend der axialen Länge des Schlitzes der ersten Hülse 4 und/oder der Schablone wird somit der Platzbedarf minimiert und eine einfache Handhabung beim Einführen des Instruments selbst bei beengten Raumverhältnissen, wie beispielsweise in der Mundhöhle, erreicht. Bevorzugt im Bereich der oberen Stirnfläche 52 ist das bereits erwähnte weitere Anschlagelement 54 vorgesehen, mit welchem das Anschlagelement der Verschiebehülse in Eingriff bzw. zur Anlage bringbar ist. Die erste Hülse 4 enthält innen eine Durchgangsbohrung mit einem Innendurchmesser 56, welcher auf den Außendurchmesser der Verschiebehülse abgestimmt und zumindest im Wesentlichen gleich groß ist wie dieser, um eine sichere Führung der Verschiebehülse in der ersten Hülse zu gewährleisten. Es sei an dieser Stelle festgehalten, dass im Rahmen der Erfindung die erste Hülse entfallen kann und das Instrument unmittelbar in einer Ausnehmung der Schablone geführt werden kann, wobei die Ausgestaltung der Innenfläche dieser Ausnehmung der Innenfläche der ersten Hülse entspricht.

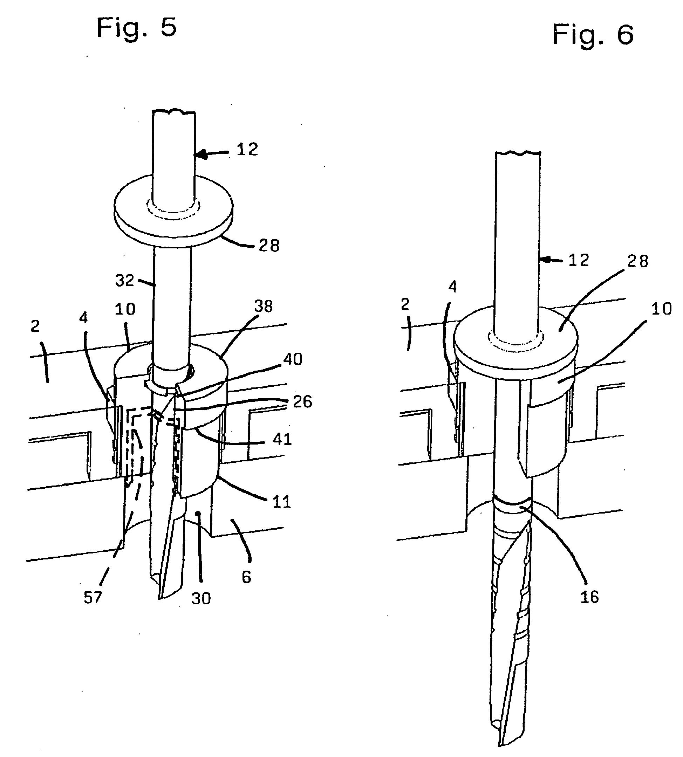

Fig. 5 und 6 zeigen weitere Phasen der Einbringung des Instruments bzw. Bohrers, wobei abweichend von Fig. 1 der Knochen nicht dargestellt ist. Gemäß Fig. 5 ist der Bohrer 12 so weit in Richtung auf die Schablone 2 und/oder die erste Hülse 4 bewegt worden, dass der erweiterte obere Randbereich 38 der Verschiebehülse 10 mit der unteren Endfläche 41 an der Schablone 2 und/oder der ersten Hülse 4 zur Anlage gebracht ist. Bei weiterer axialer Bewegung des Bohrers 12, und gleichzeitiger Drehbewegung desselben wird mittels der beiden zur Anlage bzw. in Eingriff gebrachten Anschlagelemente das Weiterdrehen der Verschiebehülse unterbunden, während die Rotation des Bohrers 12 unverändert fortgesetzt werden kann. Wie aus Fig. 6 ersichtlich, ist die Axialbewegung des Bohrers dadurch beendet, dass der Anschlag oder Ringbund 28 des Bohrers 12 zur Anlage an die obere Stirnfläche der Verschiebehülse 10 gelangt ist, welche bezüglich der Schablone 2 und/oder der ersten Hülse axial bereits festgelegt ist.

Eine alternative Ausgestaltung des Instruments 12 als Stanze ist in Fig. 5 mit gestrichelten Linien 57 dargestellt. Diese Stanze 57 ist am Schaft 32 unterhalb der Ringnut 40 in einem vorgegebenen Abstand angeordnet. Die Stanze 57 ist topfförmig und nach unten in Richtung zur Schleimhaut 6 offen ausgebildet. Der gemäß Fig. 5 nach unten anschließende Teil des Bohrers entfällt hierbei. Nach dem Lösen der Verbindung der Verschiebehülse 10 von dem derart ausgebildeten Instrument 12 wird mittels der Stanze 57 die Öffnung 30 in die Schleimhaut 6 eingebracht.

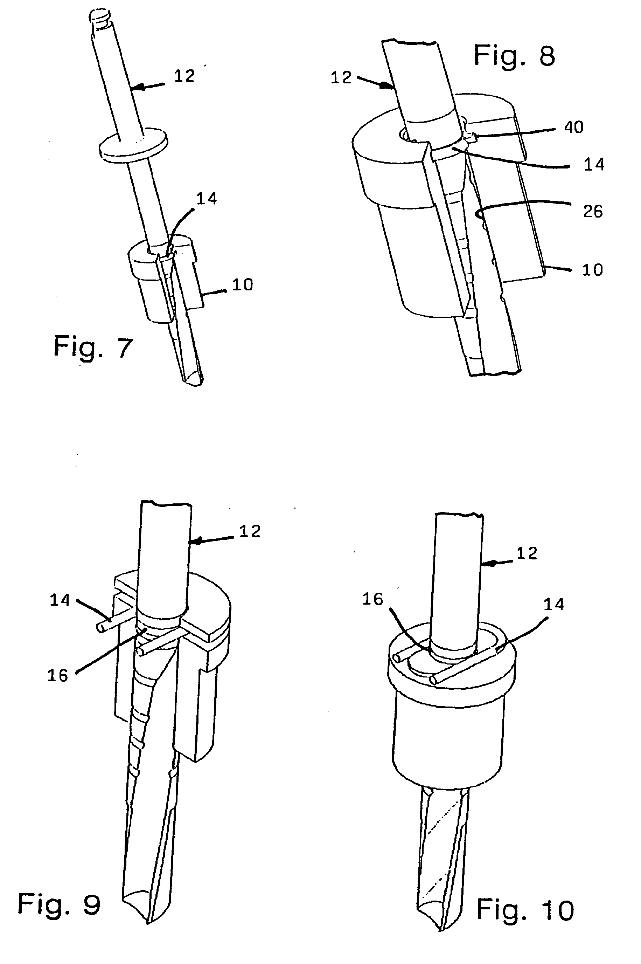

Fig. 7 und 8 zeigen perspektivisch das als Bohrer ausgebildete Instrument 12 zusammen mit der Verschiebehülse 10. Das in der Halte- oder Ringnut 40 der Verschiebehülse 10 angeordnete erste Verbindungselement 14 ist hierbei als ein geschlitzter Federring ausgebildet. Der Federring 14 greift mit einem radial innenliegenden Flächenbereich teilweise in das als Ringnut ausgebildete zweite Verbindungselement des Bohrers 2 ein. Bei Überschreiten einer vorgegebenen Axialkraft des Bohrers 12 bezüglich der Verschiebehülse 10 wird der Federring 14 aus der Ringnut bzw. dem zweiten Verbindungselement herausgedrückt und somit die Verbindung gelöst.

Fig. 9 und 10 zeigen teilweise geschnitten bzw. in perspektivischer Darstellung weitere Ausführungsbeispiele der lösbaren Verbindung, wobei das erste Verbindungselement 14 als eine Klippfeder ausgebildet ist. Die Klippfeder greift mit ihren Innenflächenbereichen in das zweite, wiederum als Ringnut ausgebildete Verbindungselement 16 des Bohrers 12 teilweise ein.

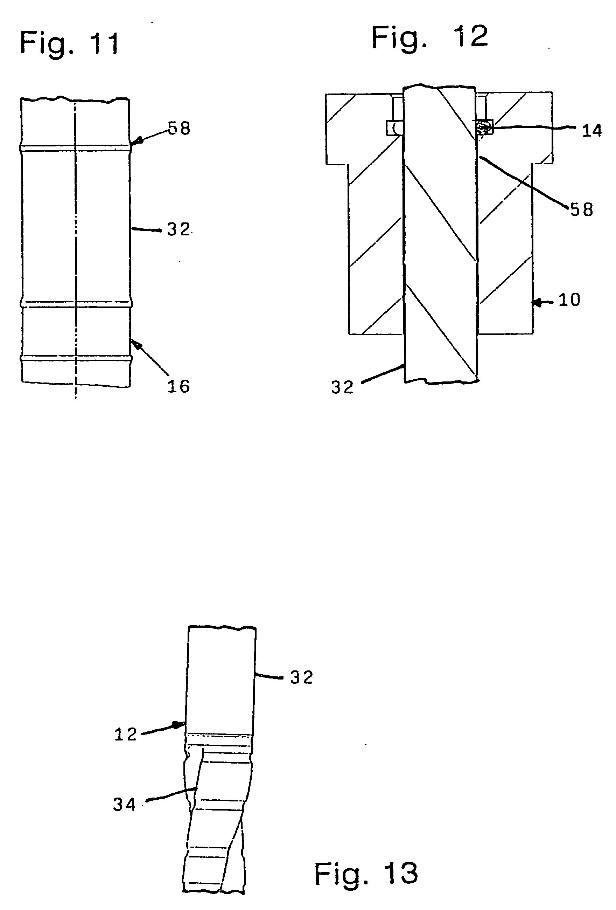

In Fig. 11 und 12 ist ein weiteres Ausführungsbeispiel zur lösbaren Festlegung der Verschiebehülse 10, am Schaft 32 des Bohrers dargestellt. Der Schaft 32 enthält für die Verschiebehülse 10 sowohl eine zweckmäßig ringförmige Führungsnocke 60 als auch das als Haltenut ausgebildete zweite Verbindungselement 16. Da die Haltenut 16 eine im Vergleich mit dem ersten Verbindungselement 14 bzw. Federelement größere axiale Längserstreckung aufweist, ist die Verschiebehülse auch bei nicht freigegebener Verbindung in einem gewissen Maß bezüglich des Bohrers 12 axial verschiebbar. Aufgrund des oder der Führungsnocken 58 wird in besonders zweckmäßiger Weise eine leicht gängige Bewegbarkeit der Verschiebehülse 10 auf dem Bohrer 12 sichergestellt.

Schließlich zeigt Fig. 13 eine Ansicht einer besonderen Geometrie des Bohrerschafts 32. Das zweite Verbindungselement 12, welches auch hier als eine Ringnut ausgebildet ist, ist zwischen dem zweckmäßig zylindrischen Schaft 32 und dem Schneidbereich 34 angeordnet.

### Bezugszeichen

- 2: Schablone
- 4: erste Hülse
- 5: durchgehende Ausnehmung in 2 oder 4
- 6: Schleimhaut / Mucosa
- 8: Knochen
- 10: zweite Hülse / Verschiebehülse
- 11: Ende von 10
- 12: Instrument / Bohrer
- 14: erstes, lösbares Verbindungselement von 10
- 16: zweites Verbindungselement von 12
- 17: Außenfläche von 10
- 18: Spitze von 12
- 20: Oberfläche von 8
- 21: Länge von 18
- 22: Pfeil / axiale Kraft
- 23: Durchgangsbohrung in 10
- 24: Außenfläche von 12
- 26: Innenfläche von 10
- 28: Anschlag / Ringbund von 12
- 30: Öffnung in 6
- 31: strichpunktierte Linie
- 32: Schaft von 12
- 34: Schneidbereich von 12
- 38: oberer Endbereich von 26
- 40: Halte- oder Ringnut in 26
- 41: untere Endfläche von 38 / Anlagefläche
- 42: strichpunktierte Linie / konischer Flächenbereich
- 43: Innendurchmesser von 10
- 44: Anschlagelement von 10
- 46: Außendurchmesser von 10
- 48: oberer Innendurchmesser von 10
- 50: Schlitz in 4
- 52: obere Stirnfläche von 4
- 54: weiteres Anschlagelement
- 56: Innendurchmesser von 4
- 57: gestrichelte Linie / Stanze
- 58: Führungsnocke

## Patentansprüche

1. Anordnung mit einem Instrument zur Vorbereitung oder Durchführung der Insertion eines Implantats, insbesondere Dentalimplantats, enthaltend eine Schablone (2), welche auf einen Knochen (8) oder die diesen umgebende Haut, insbesondere Schleimhaut, auflegbar ist, wobei die Schablone (2) oder eine in der Schablone (2) angeordnete erste Hülse (4) eine durchgehende Ausnehmung (5) aufweisen, durch welche das Instrument (12) zumindest teilweise hindurchführbar ist,
**dadurch gekennzeichnet, dass** auf dem Instrument (12) eine axial verschiebbare zweite Hülse (10) angeordnet ist, welche in einer Grundposition mittels als ineinander greifende Rastelemente ausgebildeten und selbsttätig lösbaren Verbindungselementen (14, 16) mit vorgegebener Haltekraft axial festgelegt ist,
dass die zweite Hülse (10) eine Anlagefläche (41) aufweist, welche mit der Schablone (2) und/oder der ersten Hülse (4) zur Anlage bringbar ist, wobei bei Anlage an der Schablone (2) und/oder der ersten Hülse (4) die Haltekraft überschritten werden kann,
dass bei Überschreiten der Haltekraft die Verbindungselemente (14, 16) selbsttätig lösbar sind und die zweite Hülse (10) auf dem Instrument (12) axial verschiebbar ist und
dass das Instrument (12) zusammen mit der axial festgelegten zweiten Hülse (10) in die durchgehende Ausnehmung (5) einführbar ist und Ober die an die Innenfläche der Ausnehmung (5) angepasste Außenfläche (17) der zweiten Hülse (10) in der Schablone (2) geführt und/oder ausgerichtet ist.

2. Anordnung nach Anspruch 1, **dadurch gekennzeichnet, dass** nach einem zumindest teilweise Einführen des Instruments (12) mit dem Bereich seiner Spitze (18) in die Ausnehmung (5) mittels der auf dem Instrument (12) in der Grundposition festgelegten zweiten Hülse (10) bezüglich der Schablone (2) und/ oder der ersten Hülse (4) über die an der Innenfläche der Ausnehmung (5) angepasste Außenfläche (17) der zweiten Hülse (10) eine Ausrichtung und Führung des Instruments (12) vorgegeben ist, wobei eine axiale Bewegung des Instruments (32) zusammen mit der festgelegten Hülse (10) durchführbar ist.

3. Anordnung nach Anspruch 1 oder 2, **dadurch gekennzeichnet, dass** der Außendurchmesser des Instruments (12) wesentlich kleiner ist als der Innendurchmesser (56) der ersten Hülse (4) und/oder der durchgehenden Ausnehmung der Schablone (2), und/oder dass die erste Hülse (4) in die Schablone (2) integriert ist und/oder dass die erste Hülse (4), insbesondere durch unrunde Ausbildung der Außenkontur drehgesichert in der Schablone (2) ist.

4. Anordnung nach einem der Ansprüche 1 bis 3, **dadurch gekennzeichnet, dass** das Instrument (12) in der auf diesem verschiebbaren Hülse (10) axial geführt ist, wobei die Außenfläche (24) des Instruments (12) und die Innenfläche (26) der verschiebbaren Hülse (10) bevorzugt nach Art einer Schiebepassung und/oder mit geringem und/oder mit gegen Null gehendem Spiel aneinander angepasst sind.

5. Anordnung nach einem der Ansprüche 1 bis 4, **dadurch gekennzeichnet, dass** die Außenfläche (17) der verschiebbaren Hülse (10) und die zugeordnete Innenfläche der ersten Hülse (4) oder der Schablone (2) bevorzugt nach Art einer Schiebepassung und/ oder gegen Null gehendem Spiel aneinander angepasst sind.

6. Anordnung nach einem der Ansprüche 1 bis 5, **dadurch gekennzeichnet, dass** nach Freigabe der lösbaren Verbindung (14, 16) zur Begrenzung der axialen Relativbewegung des Instruments (12) bezüglich der verschiebbaren Hülse (19) ein Anschlag (28) vorgesehen ist, welcher bevorzugt am Instrument (12) angeordnet ist.

7. Anordnung nach einem der Ansprüche 1 bis 6, **dadurch gekennzeichnet, dass** die Schablone (2) und/oder die erste Hülse (4) miteinander korrespondierende Anschlagelemente (44, 54) aufweisen, mittels welchen insbesondere eine Rotationssicherung der verschiebbaren zweiten Hülse (10) bezüglich der Schablone (2) und/oder der integrierten ersten Hülse (4) vorgegeben ist.

8. Anordnung nach Anspruch 7, **dadurch gekennzeichnet, dass** mittels der Anschlagelemente (44, 54) eine Rotationssicherung der verschiebbaren zweiten Hülse (10) bezüglich der Schablone (2) und/oder der integrierten ersten Hülse (4) vorgegeben ist.

9. Anordnung nach einem der Ansprüche 1 bis 8, **dadurch gekennzeichnet, dass** die Schablone (2) und/oder die integrierte Hülse (4) einen seitlichen Schlitz (50) aufweisen, durch welchen zumindest der Bereich der Spitze (18) des Instruments (12) hindurchführbar ist.

10. Anordnung nach einem der Ansprüche 1 bis 9, **dadurch gekennzeichnet, dass** die Verbindungselemente (14, 16) eine nach Überwindung einer vorgebbaren Kraft lösbare Verbindung, insbesondere Clippsverbindung bilden, und/oder dass die Verbindungselemente (14, 16) als lösbare Rastelemente ausgebildet sind.

11. Anordnung nach einem der Ansprüche 1 bis 10, **dadurch gekennzeichnet, dass** die Spitze (18) des Instruments (12) in einer vorgegebenen Länge (21) in der festgelegten Grundposition der verschiebbaren Hülse (10) aus dieser heraus ragt.

## Claims

1. An arrangement with an instrument for preparing or for inserting an implant, in particular a dental implant, containing a template (2) which can be placed on a bone (8) or membrane surrounding it, in particular mucous membrane, wherein the template (2) or a first sleeve (4) disposed in the template (2) comprises a through recess (5) through which at least part of the instrument (12) can pass,
**characterized in that** an axially displaceable second sleeve (10) is disposed on the instrument (12) which, in a basic position, is axially fixed with a predetermined retaining force by means of automatically releasable connecting elements (14, 16) configured as interlocking latching elements,
**in that** the second sleeve (10) exhibits a contact surface (41) which, with the template (2) and/or the first sleeve (4), can be brought into a position whereby the retaining force can be exceeded upon contact with the template (2) and/or the first sleeve (4),
**in that** the connecting elements (14, 16) are automatically releasable when the retaining force is exceeded and the second sleeve (10) is axially displaceable on the instrument (12), and
**in that** the instrument (12) together with the axially fixed second sleeve (10) can be inserted into the through recess (5) and be guided and/or orientated in the template (2) via the exterior surface (17) of the second sleeve (10) which is adapted to the interior surface of the recess (5).

2. The arrangement as claimed in claim 1, **characterized in that** the basic position of the second sleeve (10) is predetermined in a manner such that after a tip region (18) of the instrument (12) has been at least partially introduced into the recess (5) by means of the second sleeve (10) fixed in the basic position on the instrument (12) with respect to the template (2) and/or the first sleeve (4) via the exterior surface (17) of the second sleeve (10) which is adapted to the interior surface of the recess (5), orientation and guiding of the instrument (12) is provided, whereby an axial movement of the instrument (32) together with the fixed sleeve (10) can be carried out.

3. The arrangement as claimed in claim 1 or claim 2, **characterized in that** the external diameter of the instrument (12) is substantially smaller than the internal diameter (56) of the first sleeve (4) and/or the through recess of the template (2), and/or **in that** the first sleeve (4) is integrated into the template (2), and/or **in that** the first sleeve (4) is secured against rotation in the template (2), in particular by means of a non-circular configuration of the exterior contour.

4. The arrangement as claimed in one of claims 1 to 3, **characterized in that** the instrument (12) is axially guided in the sleeve (10) which is displaceable thereon, wherein the exterior surface (24) of the instrument (12) and the interior surface (26) of the displaceable sleeve (10) are preferably fitted together in the manner of a push fitting and/or with a slight or near-zero tolerance.

5. The arrangement as claimed in one of claims 1 to 4, **characterized in that** the exterior surface (17) of the displaceable sleeve (10) and the associated interior surface of the first sleeve (4) or the template (2) are preferably fitted together in the manner of a push fitting and/or with a slight or near-zero tolerance.

6. The arrangement as claimed in one of claims 1 to 5, **characterized in that** a stop (28) is provided, preferably on the instrument (12), in order to limit the relative axial movement of the instrument (12) with respect to the displaceable sleeve (19) after the releasable connection (14, 16) has been released.

7. The arrangement as claimed in one of claims 1 to 6, **characterized in that** the template (2) and/or the first sleeve (4) comprise mutually corresponding stop elements (44, 54) by means of which in particular the displaceable second sleeve (10) is secured against rotation with respect to the template (2) and/or the integrated first sleeve (4).

8. The arrangement as claimed in claim 7, **characterized in that** the stop elements (44, 54) secure the displaceable second sleeve (10) against rotation with respect to the template (2) and/or the integrated first sleeve (4).

9. The arrangement as claimed in one of claims 1 to 8, **characterized in that** the template (2) and/or the integrated sleeve (4) comprises a lateral slot (50) through which at least the tip region (18) of the instrument (12) can be passed.

10. The arrangement as claimed in one of claims 1 to 9, **characterized in that** the connecting elements (14, 16) produce a connection, in particular a snap fit connection, which can be released after exceeding a predetermined force, and/or **in that** the connecting elements (14, 16) are configured as releasable latching elements.

11. The arrangement as claimed in one of claims 1 to 10, **characterized in that** in the fixed basic position, the tip (18) of the instrument (12) protrudes out of the displaceable sleeve (10) by a predetermined length (21).

## Revendications

1. Système comportant un instrument pour la préparation ou la réalisation de l'insertion d'un implant, en particulier d'un implant dentaire, contenant un pochoir (2) qui peut être posé sur un os (8) ou dans la peau entourant celui-ci, en particulier la muqueuse, le pochoir (2) ou un premier tube (4) disposé dans le pochoir (2) présentant un premier évidement continu (5) à travers lequel l'instrument (12) peut être passé du moins partiellement,
**caractérisé en ce qu'**est disposé sur l'instrument (12) un second tube déplaçable axialement (10), qui est fixé axialement dans une position de base au moyen d'éléments de connexion (14, 16) se présentant sous forme d'éléments d'enclenchement s'engrenant les uns dans les autres et automatiquement dissociables, par une force de retenue prédéfinie,
que le second tube (10) présente une surface d'appui (41) qui peut être amenée en contact avec le pochoir (2) et/ou le premier tube (4), sachant que, en cas de contact avec le pochoir (2) et/ou le premier tube (4), la force de retenue peut être dépassée, que, en cas de dépassement de la force de retenue, les éléments de connexion (14, 16) sont automatiquement dissociables et que le second tube (10) est déplaçable axialement sur l'instrument (12) et
que l'instrument (12) peut être introduit avec le second tube fixé axialement (10) dans l'évidement continu (5) et est guidé dans et/ou aligné sur la surface extérieure (17) adaptée à la surface intérieure de l'évidement (5) du second tube (10) dans le pochoir (2).

2. Système selon la revendication 1, **caractérisé en ce que**, après une introduction au moins partielle de l'instrument (12) par la zone de sa pointe (18) dans l'évidement (5), au moyen du second tube (10) fixé en position de base sur l'instrument (12), par rapport au pochoir (2) et/ou au premier tubes (4), via la surface extérieure (17) adaptée à la surface intérieure de l'évidement (5) du second tube (10), un alignement et un guidage de l'instrument (12) sont prédéfinis, un mouvement axial de l'instrument (32) avec le tube fixé (10) pouvant être réalisé.

3. Système selon la revendication 1 ou 2, **caractérisé en ce que** le diamètre extérieur de l'instrument (12) est sensiblement inférieur au diamètre intérieur (56) du premier tube (4) et/ou de l'évidement continu du pochoir (2) et/ou que le premier tube (4) est intégré dans le pochoir (2) et/ou que le premier tube (4) se trouve bloqué en rotation dans le pochoir (2), en particulier du fait de la conformation non ronde du contour extérieur.

4. Système selon une des revendications 1 à 3, **caractérisé en ce que** l'instrument (12) est guidé axialement dans le tube (10) déplaçable sur celui-ci, la surface extérieure (24) de l'instrument (12) et la surface intérieure (26) du tube déplaçable (10) étant de préférence adaptées l'une à l'autre à la manière d'un ajustement par poussée et/ou d'un jeu faible et/ou tendant vers zéro.

5. Système selon une des revendications 1 à 4, **caractérisé en ce que** la surface extérieure (17) du tube déplaçable (10) et la surface intérieure associée du premier tube (4) ou du pochoir (2) sont de préférence adaptées l'une à l'autre à la manière d'un ajustement par poussée et/ou d'un jeu faible et/ou tendant vers zéro.

6. Système selon une des revendications 1 à 5, **caractérisé en ce que**, après le déblocage de la connexion dissociable (14, 16), pour limiter le mouvement relatif axial de l'instrument (12) par rapport au tube déplaçable (19), il est prévu une butée (28) qui est disposée de préférence au niveau de l'instrument (12).

7. Système selon une des revendications 1 à 6, **caractérisé en ce que** le pochoir (2) et/ou le premier manchon (4) présentent des éléments de butée (44, 54) se correspondant mutuellement au moyen desquels en particulier un blocage de rotation du second manchon déplaçable (10) par rapport au pochoir (2) et/ou au premier tube intégré (4) est prédéfini.

8. Système selon la revendication 7, **caractérisé en ce que**, au moyen des éléments de butée (44, 54), un blocage de rotation du second manchon déplaçable (10) par rapport au pochoir (2) et/ou au premier tube intégré (4) est prédéfini.

9. Système selon une des revendications 1 à 8, **caractérisé en ce que** le pochoir (2) et/ou le tube intégré (4) présentent une fente latérale (50) à travers laquelle au moins la zone de la pointe (18) de l'instrument (12) peut être passée.

10. Système selon une des revendications 1 à 9, **caractérisé en ce que** les éléments de connexion (14, 16) établissent une connexion dissociable après avoir surmonté une force prédéfinissable, en particulier une connexion par clipsage et/ou que les éléments de connexion (14, 16) se présente sous forme d'éléments d'enclenchement dissociables.

11. Système selon une des revendications 1 à 10, **caractérisé en ce que** la pointe (18) de l'instrument (12) d'une longueur prédéfinie (21), en position de base fixée du tube déplaçable (10), dépasse de celui-ci.
